# EUROPEAN PATENT APPLICATION

(11) **EP 2 781 215 A1**
(43) Date of publication of application: **24.09.2014**
(21) Application number: 12850012.1
(22) Date of filing: 19.11.2012
(51) Int. Cl.: A61K 9/70, A61P 17/00, A61F 5/00

(54) **TOPICAL PATCH PREPARATION FOR TREATMENT OF TOXICITY OF MOSQUITOES**

(30) Priority: 17.11.2011 KR 20110120254
(71) Applicant: Lim, Hae Eun, Incheon 405-300 (KR)
(72) Inventor: Lim, Hae Eun, Incheon 405-300 (KR)
(74) Representative: Leske, Thomas
(86) International application number: PCT/KR2012/009804
(87) International publication number: WO 2013/073926

(57) **Abstract**

A topical patch preparation for treatment of toxicity of mosquitoes proposed by the present invention includes: a back sheet (100) and an adhesive heating element (200) stacked and attached to the lower surface of the back sheet (100) and including exothermic compositions mixed therein to generate heat. Thus, the topical patch preparation may decompose the toxicity of mosquitoes caused by a mosquito bite simply by using heat without chemicals or drugs so as to immediately relieve itching and swelling.

## Description

### [TECHNICAL FIELD]

The present invention relates to a therapeutic agent for toxicity of mosquitoes, and more particularly, to a topical patch preparation for treatment of toxicity of mosquitoes that can decompose toxicity of mosquitoes without the addition of chemicals or drugs.

### [BACKGROUND ART]

Mosquitoes are harmful insects which cause lethal diseases such as malaria, encephalitis, yellow fever, dengue fever, and the like as well as directly or indirectly damaging people in a way such as sleeping disturbance, restrictions on outdoor activities, and the like resulting from bloodsucking people and livestock, and are rampant particularly in the hot summer season. A variety of products for eradicating mosquitoes are now manufactured and sold, but human bodies cannot be fully protected from mosquitoes even when using such products and there is still a risk of mosquito bites.

If bitten by a mosquito, the bitten body part may be itching and symptoms of swelling red may be expressed. More specifically, when the mosquito's saliva penetrates the surface of the skin and capillaries, blood is naturally sucked into the mosquito's mouth due to high blood pressure in the capillaries. In order to prevent the clotting of blood during bloodsucking of mosquitoes, a substance named hirudin of the mosquito enters the human body, and a substance named histamine may be secreted inside the human body as a defense mechanism against this. Histamine normally stays in an inactive state in conjunction with tissues, but is secreted immediately when foreign substances such as hirudin of the mosquito intrude into the human body, and therefore widens capillaries near the intrusion. As a result, a large amount of the immune system acting in blood are moved to the body part bitten by the mosquito, and therefore the surface of the skin near the body part bitten by the mosquito is itchy, swollen, and red. In addition, when such histamine is explosively increased to cause panic, the whole body is swollen, and when it spreads to the inside of the body to block the airway, suffocation may occur.

Therefore, in order to relieve the itching and the swelling by decomposing the mosquito-toxic components, a variety of therapeutic agent products such as antihistamine in liquid formulations which can be applied on the surface of the skin have been manufactured and sold (see, Korean Patent Publication No. 10-2004-0002823). However, when applied to areas of the skin bitten by the mosquito, such products may be easily evaporated to cause loss of their active ingredients. In addition, such products contain various chemicals and drugs as the active ingredients and these are applied to the surface of the skin, and therefore side effects causing skin irritation may occur in the case of patients with sensitive skin such as children. Meanwhile, a primitive method such as using a cold pack, temporarily applying saliva, or pressing with fingernails is used, but it cannot be referred to a method of actually decomposing the toxicity of mosquitoes.

As described above, development of therapeutic agents which can relieve itching and swelling by decomposing impregnated mosquito's toxicity without the use of chemicals or drugs has become an important task.

### [DISCLOSURE]

### [TECHNICAL PROBLEM TO BE SOLVED]

The present invention is directed to providing a topical patch preparation for treatment of toxicity of mosquitoes that may include a back sheet 100 and an adhesive heating element 200 stacked and attached to the lower surface of the back sheet 100 and including exothermic compositions mixed therein to generate heat. Thus, the topical patch preparation may decompose the toxicity of mosquitoes caused by a mosquito bite simply by using heat without chemicals or drugs so as to immediately relieve itching and swelling.

### [TECHNICAL SOLUTION]

One aspect of the present invention provides a topical patch preparation for treatment of toxicity of mosquitoes, including: a back sheet (100); and an adhesive heating element (200) stacked and attached to a lower surface of the back sheet (100) and including exothermic compositions mixed therein to generate heat.

Preferably, the topical patch preparation may further include a protective film (300) attached to a lower surface of the adhesive heating element (200) to protect the adhesive heating element (200).

Preferably, the exothermic compositions may contain iron (Fe₂O₃), activated carbon, salt (NaCl), vermiculite, and sodium acetate.

Preferably, the adhesive heating element (200) may further include a water-soluble polymer material, water, and a moisture preserver.

Preferably, the topical patch preparation may be vacuum-packaged to be easily carried.

Preferably, the back sheet (100) may be made of a material such as a non-woven fabric or cotton.

### [ADVANTAGEOUS EFFECTS]

According to embodiments of the present invention, there is provided a topical patch preparation for treatment of toxicity of mosquitoes that may include a back sheet 100 and an adhesive heating element 200 stacked and attached to the lower surface of the back sheet 100 and including exothermic compositions mixed therein to generate heat. Thus, the topical patch preparation may decompose the toxicity of mosquitoes caused by a mosquito bite simply by using heat without chemicals or drugs so as to immediately relieve itching and swelling.

### [DESCRIPTION OF DRAWINGS]

FIG. 1 is an exploded perspective view showing a topical patch preparation 10 for treatment of toxicity of mosquitoes according to an embodiment of the present invention.
FIG. 2 is a perspective view showing a topical patch preparation 10 for treatment of toxicity of mosquitoes according to an embodiment of the present invention.
FIG. 3 is a cross-sectional view showing a topical patch preparation 10 for treatment of toxicity of mosquitoes according to an embodiment of the present invention.
FIG. 4 is a view showing a state in which a topical patch preparation 10 for treatment of toxicity of mosquitoes according to an embodiment of the present invention is drawn out of a package container in which the topical patch preparation 10 is vacuum-packaged.
FIG. 5 is a view showing a state in which a topical patch preparation 10 for treatment of toxicity of mosquitoes according to an embodiment of the present invention is attached to a topical area.
FIG. 6 is a cross-sectional view showing a state in which a topical patch preparation 10 for treatment of toxicity of mosquitoes according to an embodiment of the present invention is attached to a topical area.

### [BEST MODE]

Hereinafter, exemplary embodiments of the present invention will be described in detail. However, the present invention is not limited to the exemplary embodiments disclosed below, but can be implemented in various forms. The following exemplary embodiments are described in order to enable those of ordinary skill in the art to embody and practice the invention. When it is determined that the detailed description of known art related to the present invention may obscure the gist of the present invention, the detailed description thereof will be omitted. The same reference numerals are used to refer to the same element throughout the specification.

It will be understood that when an element is referred to as being "connected to" another element, it can be "directly connected to" the other element or intervening elements may be present. In addition, it will be further understood that the terms "comprises" and/or "comprising," when used in this specification, specify the presence of stated features, integers, steps, operations, elements, and/or components, but do not preclude the presence or addition of one or more other features, integers, steps, operations, elements, components, and/or groups thereof.

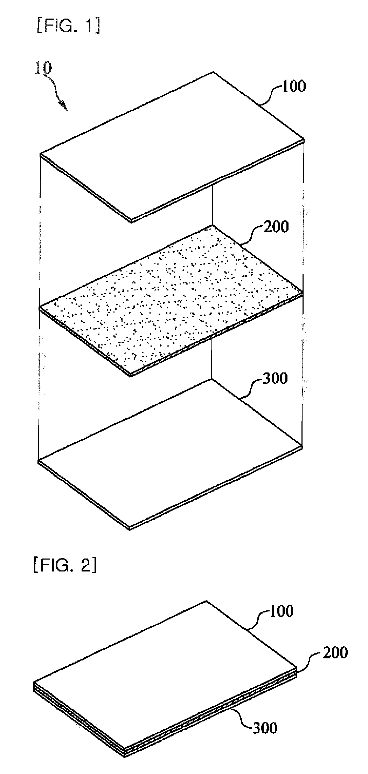

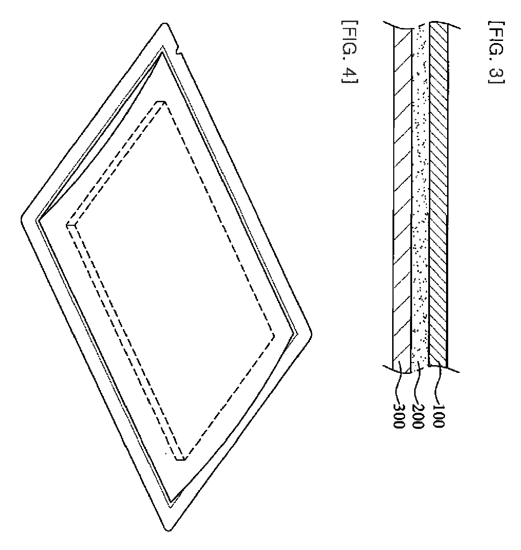

FIG. 1 is an exploded perspective view showing a topical patch preparation 10 for treatment of toxicity of mosquitoes according to an embodiment of the present invention, FIG. 2 is a perspective view showing a topical patch preparation 10 for treatment of toxicity of mosquitoes according to an embodiment of the present invention, and FIG. 3 is a cross-sectional view showing a topical patch preparation 10 for treatment of toxicity of mosquitoes according to an embodiment of the present invention. As shown in FIGS. 1 to 3, the topical patch preparation 10 may include a back sheet 100 and an adhesive heating element 200 that is stacked and attached to a lower surface of the back sheet 100, and further include a protective film 300.

The back sheet 100 supports the adhesive heating element 200 which will be described later and fixes the adhesive heating element 200 at the body part bitten by a mosquito when the human body moves in a state in which the topical patch preparation 10 is attached to the body part bitten by the mosquito, and is preferably made of a textile material such as well-ventilated cotton. More preferably, the back sheet 100 may be prepared using a non-woven fabric, and a bulky non-woven fabric can be used for good feeling. In addition, for convenience of description, the back sheet 100 has a rectangular shape, but the shape of the back sheet 100 is not limited thereto. The back sheet 100 may have various shapes such as a triangle, a circle, and the like, which can be appropriately implemented by those skilled in the art.

The adhesive heating element 200 is stacked and attached to the lower surface of the back sheet 100 to generate heat, thereby relieving itching and swelling by decomposing impregnated mosquito's toxicity. The adhesive heating element 200 may include exothermic compositions. More specifically, the exothermic compositions containing iron (Fe₂O₃), activated carbon, salt (NaCl), vermiculite, and sodium acetate may exist while being dispersed in the adhesive heating element 200, and an amount of the exothermic compositions included in the adhesive heating element 200 may be included as an amount of the exothermic compositions capable of generating sufficient heat when attaching the topical patch preparation 10 according to an embodiment of the present invention to the surface of the skin, which is appropriately selected and implemented by those skilled in the art.

As each component contained in the exothermic compositions, iron (Fe₂O₃) and activated carbon generate heat through reaction with air, whereby a temperature of the surface of the skin bitten by a mosquito may be raised up to 40 to 60°C. Vermiculite has an excellent energy-absorbing effect to thereby effectively absorb the heat generated from iron (Fe₂O₃) and activated carbon, and sodium acetate retains the heat generated from iron (Fe₂O₃) and activated carbon to thereby continuously maintain the temperature of 40 to 60°C. In addition, salt (NaCl) may allow reaction of iron (Fe₂O₃), activated carbon, and air to be rapidly achieved, thereby generating heat at the same time when the topical patch preparation 10 according to an embodiment of the present invention is attached to the surface of the skin bitten by a mosquito.

In addition, the adhesive heating element 200 itself has adhesive properties, and thereby may be attached to the surface of the skin bitten by the mosquito without the use of an auxiliary adhesive such as an adhesive tape or the like.

In this manner, the topical patch preparation 10 according to an embodiment of the present invention includes the exothermic compositions capable of generating heat, whereby itching or swelling may be immediately relieved by decomposing the impregnated mosquito's toxicity simply using heat without the addition of any chemical substance or drug for relieving itching and swelling by decomposing the toxicity of mosquito.

Meanwhile, the adhesive heating element 200 may further include a water-soluble polymer material, water, and a moisture preserver. A water-soluble polymer may be included in the water-soluble polymer material, and the polymer may be gelatin, starch, agar, mannan, alginic acid, polyacrylic acid, sodium polyacrylate, dextrin, methyl cellulose, sodium methyl cellulose, hydroxypropyl cellulose, carboxymethylcellulose, cellulose gum, a carboxyvinyl polymer, polyvinyl alcohol, polyvinylpyrrolidone, or a mixture of these, but is not limited thereto. In addition, the water may allow reaction of iron (Fe₂O₃), activated carbon, and air in the adhesive heating element 200 to be rapidly achieved or impart other desirable physical properties, and therefore normal water is preferably used, but the present invention is not limited thereto. For example, distilled water or ion-exchange water may be used. The moisture preserver is a component for allowing water contained in the adhesive heating element 200 to be maintained substantially constant during storage or use of the topical patch preparation 10 according to an embodiment of the present invention by minimally reducing volatilization of water contained in the adhesive heating element 200, and may be glycerin, sorbitol, propylene glycol, diethylene glycol, 1,3-butylene glycol, ethylene glycol, or the like, but is not limited thereto.

The protective film 300 is attached to a lower surface of the adhesive heating element 200 to protect the adhesive heating element 200 from an ambient environment, and preferably made of a synthetic material such as polyethyleneterephthalate (PET), polypropylene (PP), or the like, but is not limited thereto. That is, the protective film 300 maintains adhesive properties of the adhesive heating element 200 while protecting the adhesive heating element 200, and may be easily removed from the adhesive heating element 200 when using the topical patch preparation 10 according to an embodiment of the present invention.

In addition, the topical patch preparation 10 for treatment of toxicity of mosquitoes according to an embodiment of the present invention is preferably vacuum-packaged so as to be easily carried. FIG. 4 is a view showing a state in which the topical patch preparation 10 for treatment of toxicity of mosquitoes according to an embodiment of the present invention is drawn out of a package container in which the topical patch preparation 10 is vacuum-packaged. As shown in FIG. 4, the topical patch preparation 10 for treatment of toxicity of mosquitoes according to an embodiment of the present invention may be received into a package container made of a vinyl material to be sealed in a vacuum state, and the package container may further include a layer through which moisture, oxygen, and other substances cannot pass. When using the topical patch preparation 10 for treatment of toxicity of mosquitoes according to an embodiment of the present invention, the package container may be opened through an opening line so as to take the topical patch preparation 10 out of the package container, and then the topical patch preparation 10 may be attached to the body part bitten by the mosquito.

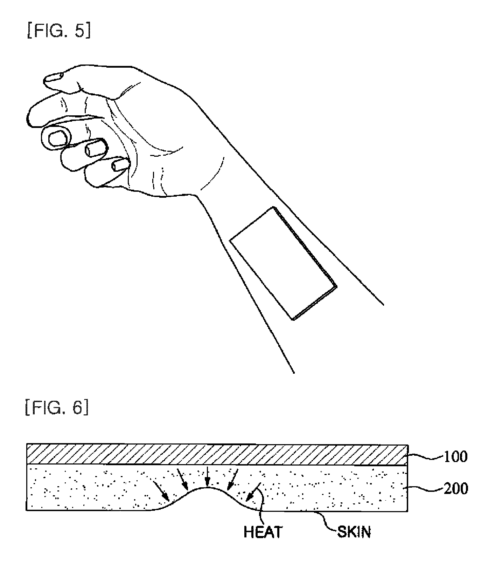

FIG. 5 is a view showing a state in which the topical patch preparation 10 for treatment of toxicity of mosquitoes according to an embodiment of the present invention is attached to a topical area, and FIG. 6 is a cross-sectional view showing a state in which the topical patch preparation 10 for treatment of toxicity of mosquitoes according to an embodiment of the present invention is attached to a topical area. As shown in FIG. 5, the topical patch preparation 10 for treatment of toxicity of mosquitoes according to an embodiment of the present invention may be attached to an arbitrary topical area bitten by a mosquito. An area of the surface of the skin covered by the topical patch preparation 10 for treatment of toxicity of mosquitoes according to an embodiment of the present invention should be sufficient to transfer sufficient heat to the body part bitten by the mosquito, preferably approximately 10 to 100 cm², and more preferably approximately 5 to 30 cm².

In addition, as shown in FIG. 6, in order to attach the topical patch preparation 10 for treatment of toxicity of mosquitoes according to an embodiment of the present invention to the body part bitten by the mosquito, the adhesive heating element 200 is exposed by removing the protective film 300 from the adhesive heating element 200, and then the exposed adhesive heating element 200 is attached to the corresponding area to be used. The adhesive heating element 200 itself has adhesive properties, and therefore may be attached to the surface of the skin without the use of an auxiliary adhesive such as an adhesive tape. The exothermic compositions dispersed in the adhesive heating element 200 rapidly react with air to generate heat, and the generated heat may decompose the impregnated mosquito's toxicity to immediately relieve itching and swelling.

Hereinafter, the present invention will be described in more detail by the following Example. However, the scope of the present invention is not limited to the following Example.

### [Example 1]

### Manufacturing of topical patch preparation

An adhesive heating element including the content of each component described in Table 1 was spread on a non-woven fabric, was laminated with a PP film, and then was cut into a rectangle having a size of 5 cm, thereby obtaining a topical patch preparation.

**[Table 1]**

| Component | Content (g) |
|---|---|
| Iron (Fe₂O₃) | 28 |
| Activated carbon | 10 |
| Vermiculite | 8 |
| Salt (NaCl) | 2 |
| Sodium acetate | 2 |
| Water | 30 |
| Polyacrylic acid | 40 |
| Glycerin | 10 |
| Sorbitol | 6 |
| Total | 100 |

Hereinafter, the present invention will be described in more detail by the following Experimental Example. However, the scope of the present invention is not limited to the following Experimental Example.

### [Experimental Example 1]

### Experiment on human body concerning decomposition effect on toxicity of mosquitoes

In order to confirm the decomposition effect on toxicity of mosquitoes in the topical patch preparation obtained in Example 1, effect evaluation was performed on robust ten men in their 20s to 30s who were less than 5 hours after bitten by a mosquito.

That is, subjects were divided into an experimental group of five and a comparison group of five. Next, in the experimental group, the topical patch preparation obtained in Example 1 was attached to the body part of the subject bitten by the mosquito, and in the comparison group, a therapeutic agent for toxicity of mosquitoes in liquid formulations which was commercially available was applied. The state of the body part bitten by the mosquito was observed in 10 minutes, and the results are shown in Table 2.

**[Table 2]**

| Division | | decomposition effect on toxicity of mosquitoes | |
|---|---|---|---|
| | | (people) | (%) |
| Experimental group | Very nice | 4 | 80.0 |
| | Nice | 1 | 20.0 |
| | normal | - | - |
| | So-so | - | - |
| Comparison group | Very nice | - | - |
| | Nice | 1 | 20.0 |
| | normal | 2 | 40.0 |
| | So-so | 2 | 40.0 |

As can be seen from Table 2, it could be confirmed that, in at least 80% of the subjects to whom the topical patch preparation obtained in Example 1 was attached, the toxicity of mosquitoes was decomposed to relieve itching and swelling. Thus, it could be confirmed that the topical patch preparation according to an embodiment of the present invention was very effective in rapidly treating the toxicity of mosquitoes.

While the invention has been shown and described with reference to certain exemplary embodiments thereof, it will be understood by those skilled in the art that various changes in form and details may be made therein without departing from the spirit and scope of the invention as defined by the appended claims.

### [EXPLANATION OF REFERENCE CHARACTERS]

10: Topical patch preparation for treatment of toxicity of mosquitoes according to an embodiment of the present invention
100: Back sheet
200: Adhesive heating element
300: Protective film

## Claims

1. A topical patch preparation for treatment of toxicity of mosquitoes, comprising:
a back sheet (100); and
an adhesive heating element (200) stacked and attached to a lower surface of the back sheet (100) and including exothermic compositions mixed therein to generate heat,
wherein the exothermic compositions contain iron (Fe₂O₃), activated carbon, salt (NaCl), vermiculite, and sodium acetate, and
the adhesive heating element (200) further includes a water-soluble polymer material, water, and a moisture preserver.

2. The topical patch preparation according to claim 1, further comprising a protective film (300) attached to a lower surface of the adhesive heating element (200) to protect the adhesive heating element (200).

3. The topical patch preparation according to claim 1, wherein the topical patch preparation is vacuum-packaged to be easily carried.

4. The topical patch preparation according to claim 1, wherein the back sheet (100) is made of a material such as a non-woven fabric or cotton.
